(19) Europäisches Patentamt
European Patent Office
Office européen des brevets

(11) **EP 4 357 030 A1**

(12) **EUROPEAN PATENT APPLICATION**

(43) Date of publication:
**24.04.2024  Bulletin 2024/17**

(21) Application number: **23199270.2**

(22) Date of filing: **25.09.2023**

(51) International Patent Classification (IPC):
***B05C 17/005*** (2006.01)

(52) Cooperative Patent Classification (CPC):
**B05C 17/00516; B05C 17/00506**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **26.09.2022  JP 2022152908**

(71) Applicant: **Shofu Inc.**
**Kyoto-shi, Kyoto 605-0983 (JP)**

(72) Inventors:
• **SHIGA, Toshio**
**Tokyo, 103-8538 (JP)**

• **SASAKI, Tsukasa**
**Tokyo, 103-8538 (JP)**
• **TAKEI, Ryoji**
**Tokyo, 103-8538 (JP)**
• **NAKATSUKA, Toshiyuki**
**Kyoto, 605-0983 (JP)**
• **MIYATA, Shunsuke**
**Kyoto, 605-0983 (JP)**
• **KOBAYASHI, Hiroyuki**
**Kyoto, 605-0983 (JP)**
• **MATSUMOTO, Akiko**
**Kyoto, 605-0983 (JP)**

(74) Representative: **Eisenführ Speiser**
**Patentanwälte Rechtsanwälte PartGmbB**
**Postfach 31 02 60**
**80102 München (DE)**

(54) **SYRINGE TIP HAVING BLADE DIRECTION AND DISCHARGE DIRECTION ALIGNED WITH EACH OTHER**

(57)    To provide a syringe tip that can prevent the syringe tip from unintentionally falling-off during filling and prevent viscous material from leaking out from gaps by securely attaching the syringe tip to a syringe and can provide excellent operability without obstructing a surgical field.

To provide syringe tip to be attached to a tip of a syringe containing viscous material to discharge the viscous material, comprising
a syringe engaging portion that engages with the syringe,
a cylindrical portion having a hollow portion with a virtual center line at its center and held by a user in the case of engaging the syringe tip with the syringe, and
a nozzle portion discharging the viscous material contained in the syringe,
wherein in the case that the maximum length (mm) between both end portions of the cylindrical portion on a virtual straight line orthogonal to the virtual central line is defined as first length A, the virtual straight line orthogonal to the virtual central line and including both end portions of the cylindrical portion constituting the first length A is defined as first virtual straight line, and the length (mm) between both end portions of the cylindrical portion on a second virtual straight line orthogonal to the virtual central line and orthogonal to the first virtual straight line is defined as second length B, the first length A and the second length B satisfy the following relational expressions (1) to (3).

Relational expression (1): First length A / Second length B $\geq$ 1.5

Relational expression (2): $13 \geq$ (First length A + Second length B) $\geq 5$

Relational expression (3): $5 \geq$ Second length B

EP 4 357 030 A1

[Fig.10]

**Description**

BACKGROUND OF THE INVENTION

Field of the Invention

The present invention relates to a syringe tip that is attached to a tip of a syringe containing viscous material and discharges the viscous material.

Description of the Related Art

In the dental field, paste materials (viscous materials) such as dental filling materials and materials for preparing dental crowns may be filled into injectors such as syringe which can contain paste materials in the inside thereof in order to optimize a discharge amount and used. As such injectors, there is one in which a replaceable syringe tip is attached to a tip of a cylindrical syringe, as described in US Pat. No. 5,445,523.
The syringe tip is formed with a flow path in the inside thereof and one end of the flow path is connected to a discharge opening. The syringe tip is configured to discharge pasty material filled in the inside of the syringe from the discharge opening of the syringe tip via the flow path of the syringe tip by pushing operation of a push rod from the rear end side of the syringe.

SUMMARY OF THE INVENTION

Technical Problem

The syringe tip disclosed in US Pat. No. 5,445,523 is formed with a tab (blade) and operator can firmly attach the syringe tip to the syringe by this tab. However, in the syringe tip disclosed in US Pat. No. 5,445,523, the tab obstructs a surgical field, and therefore it is difficult to see a cavity to be filled with viscous material. In addition, because the tab is large and obstructs in the narrow oral cavity, it is impossible to provide excellent operability.
An object of the present invention is to provide a syringe tip that can prevent the syringe tip from unintentionally falling-off during filling and prevent viscous material from leaking out from gaps by securely attaching the syringe tip to a syringe and can provide excellent operability without obstructing a surgical field.

Solution to Problem

The present invention provides, as one embodiment, a syringe tip to be attached to a tip of a syringe containing viscous material to discharge the viscous material, comprising

a syringe engaging portion that engages with the syringe,
a cylindrical portion having a hollow portion with a virtual center line at its center and held by a user in the case of engaging the syringe tip with the syringe, and
a nozzle portion discharging the viscous material contained in the syringe,
wherein in the case that the maximum length (mm) between both end portions of the cylindrical portion on a virtual straight line orthogonal to the virtual central line is defined as first length A, the virtual straight line orthogonal to the virtual central line and including both end portions of the cylindrical portion constituting the first length A is defined as first virtual straight line, and the length (mm) between both end portions of the cylindrical portion on a second virtual straight line orthogonal to the virtual central line and orthogonal to the first virtual straight line is defined as second length B,
the first length A and the second length B satisfy the following relational expressions (1) to (3).

$$\text{Relational expression (1): First length A / Second length B} \geq 1.5$$

$$\text{Relational expression (2): } 13 \geq (\text{First length A} + \text{Second length B}) \geq 5$$

$$\text{Relational expression (3): } 5 \geq \text{Second length B}$$

Advantageous Effects of Invention

The present invention can provide a syringe tip that can prevent the syringe tip from unintentionally falling-off during filling and prevent viscous material from leaking out from gaps by securely attaching the syringe tip to a syringe and can provide excellent operability without obstructing a surgical field.

Brief description of drawings

[FIG. 1] Schematic view showing a configuration of set according to one embodiment of the present invention.
[FIG. 2] Front view of syringe according to one embodiment.
[FIG. 3] Plan view of syringe according to one embodiment.
[FIG. 4] Sectional view along I-I line in Figure 2.
[FIG. 5] Front view of push rod according to one embodiment.
[FIG. 6] Plan view of push rod according to one embodiment.
[FIG. 7] Sectional view along III-III line in Figure 5.
[FIG. 8] Front view of syringe tip according to one embodiment.
[FIG. 9] Plan view of syringe tip according to one embodiment.
[FIG. 10] Sectional view along II-II line in Figure 8.
[FIG. 11] Sectional view of syringe tip and syringe in the state of attaching the syringe tip to the tip of the syringe.

DETAILED DESCRIPTION OF THE PREFERRED EMBODIMENTS

In one embodiment of the present invention, the cylindrical portion may comprise a main body portion formed with the hollow portion and a pair of blade portions provided on an outer wall portion of the main body portion, the first length A may be a length between the tip portions of the pair of blade portions, and the second length B may be a length between the outer walls of the main body portion.

In one embodiment of the present invention, the nozzle portion may extend in a direction inclined with respect to the virtual central line of the hollow portion of the cylindrical portion, and the pair of blade portions may be provided parallel to an extending direction of the nozzle portion.

In one embodiment of the present invention, the First length A further may satisfy the following relational expression (4).

$$\text{Relational expression (4): First length } A \leq 8$$

In one embodiment of the present invention, in the case that an inner diameter (mm) of the nozzle portion on the discharge opening side is defined as inner diameter C, the inner diameter C may satisfy the following relational expression (5).

$$\text{Relational expression (5): } 1.50 \geq \text{Inner diameter } C \geq 0.12$$

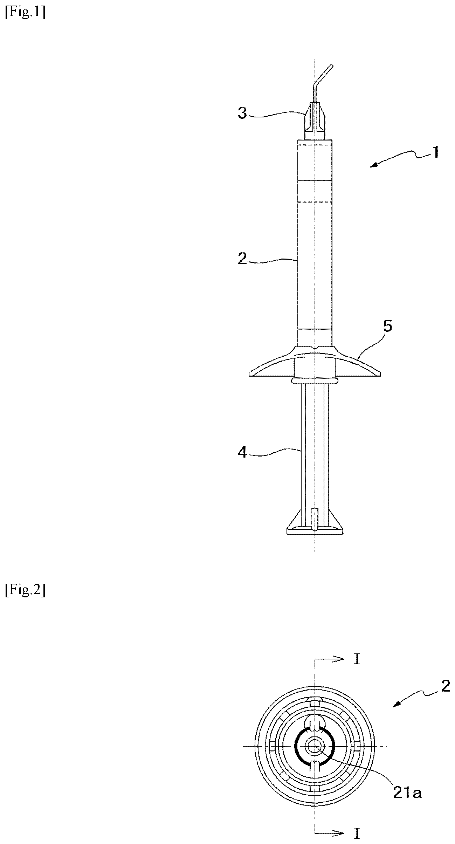

Hereinafter, specific embodiments of the present invention will be described in detail with reference to the drawings. Figure 1 is a schematic view showing a configuration of a nozzle exchangeable injector 1 according to one embodiment of the present invention. In the present embodiment, the injector 1 comprises a syringe 2 and a syringe tip 3 that is detachably and threadably attached to a tip portion 21 of the syringe 2. In the present embodiment, viscous material is contained in the inside of the syringe 2. In the present embodiment, the injector 1 constitutes the set of the present invention. The syringe tip 3 of the present embodiment is attached and fixed to the tip porion of the syringe 2 by rotating operation in a predetermined direction from the tip of the syringe 2.

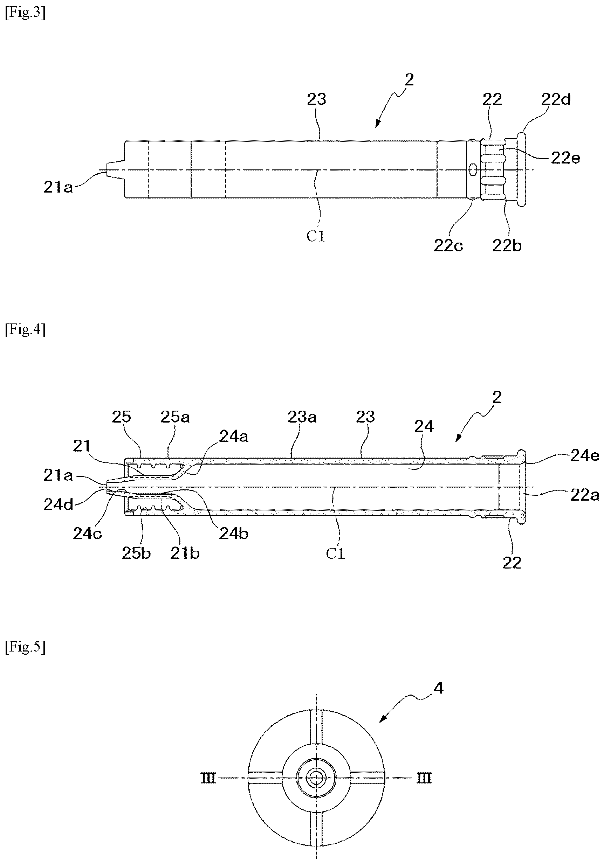

Figure 2 is a front view of syringe 2, Figure 3 is a plan view of syringe 2 and Figure 4 is a sectional view along I-I line in Figure 2. The syringe 2 is also sometimes referred to as a barrel. The syringe 2 is a cylindrical member having the center on the central axis C1, and is made of, for example, a resin material or a glass material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene- vinyl acetate copolymerization) resin, styrene resin, polymethylpentene, methacrylstyrene, ABS (acrylonitrile, butadiene, styrene) resin, polycarbonate and the like can be used. In the present embodiment, the syringe 2 made of polypropylene.

The syringe 2 of the present embodiment comprises a push rod 4. The push rod 4 is also called a plunger. The syringe 2 of the present embodiment comprises a tip portion 21 having a discharge opening 21a at one end, a rear end portion 22 having at one end an insertion opening 22a inserted with the push rod 4 described later, and the main body portion 23 positioned between the tip portion 21 and the rear end portion 22. The outer diameter of the syringe 2 may be, for example, 3 mm to 15 mm.

The tip portion 21 of the present embodiment is formed into a tapered cylindrical shape so that a cross-sectional size decreases toward the discharge opening 21a. The dimension of the tip portion 21 in the longitudinal direction of the syringe may be, for example, 1 mm to 20 mm. The main body portion 23 of the present embodiment is formed into a cylindrical shape so that a cross-sectional size is constant. The dimension of the main body portion 23 in the longitudinal direction of the syringe may be, for example, 30 mm to 150 mm. The rear end portion 22 of the present embodiment is formed with a finger grip attachment portion 22b to which a finger grip 5 is attached. The dimension of the rear end portion 22 in the longitudinal direction of the syringe may be, for example, 1 mm to 20 mm.

The finger grip attachment portion 22b of the present embodiment includes a pair of flange portions 22c and 22d formed over the entire circumferential surface of the syringe 2, and eight recess portions 22e formed between the pair of flange portions 22c and 22d. The pair of flange portions 22c and 22d restricts movement of the finger grip 5 along the longitudinal direction of the syringe 2. In the present embodiment, the flange portion 22d is formed larger than the flange portion 22c. Further, in the present embodiment, the flange portion 22d is formed at the rear end of the rear end portion 22. The eight recess portions 22e are fitted with eight protrusions (not shown) provided in the hollow portion (not shown) of the finger grip 5. Due to this fitting, rotation of the finger grip 5 in the circumferential direction of the syringe 2 is restricted.

The inside of the syringe 2 is formed with a hollow portion 24 which can contain viscous material therein over the entire length in the longitudinal direction centering on the central axis C1. The hollow portion 24 in the tip portion 21 is formed into a tapered shape so that a cross-sectional size decreases toward the discharge opening 21a. Specifically, the hollow portion 24 in the tip portion 21 includes a first tapered portion 24a, a first columnar portion 24b, a second tapered portion 24c and a second columnar portion 24d. The first tapered portion 24a has one end continuous with the main body portion 23, and is configured so that a cross-sectional size decreases from the cross-sectional size in the main body portion 23 toward the discharge opening 21a. The first columnar portion 24b has one end continuous with the first tapered portion 24a, and is configured so that a cross-sectional size is constant, for example, an inner diameter of 0.1 mm to 13 mm. The second tapered portion 24c has one end continuous with the first columnar portion 24b, and is configured so that a cross-sectional size decreases from the cross-sectional size in the first columnar portion 24b toward the discharge opening 21a. The second columnar portion 24d has one end continuous with the second tapered portion 24c and the other end continuous with the discharge opening 21a, and is configured so that a cross-sectional size is constant, for example, an inner diameter of 0.1 mm to 10 mm.

In the main body portion 23, the hollow portion 24 is formed into a cylindrical shape so that a cross-sectional size is constant, for example, an inner diameter of 0.1 mm to 13 mm. In the rear end portion 22, the hollow portion 24 is formed into a cylindrical shape so that a cross-sectional size is constant, for example, an inner diameter of 0.1 mm to 15 mm. In particular, in the present embodiment, the hollow portion 21 in the rear end portion 22 is formed with a third tapered portion 24e so that a cross-sectional size slightly increases toward the insertion opening 22a in a portion continuous with the insertion opening 22a in order to facilitate the insertion of the push rod 4.

In the syringe 2 of the present embodiment, a syringe tip mounting portion 25 is formed on the outer peripheral side of the tip portion 21. In the present embodiment, the syringe tip mounting portion 25 is circularly provided around the central axis C1. In the present embodiment, the syringe tip mounting portion 25 is integrally formed with the syringe 2.

The syringe tip mounting portion 25 of the present embodiment has the same outer diameter as the main body portion 23, and the outer peripheral surface 25a of the syringe tip mounting portion 25 is configured to be substantially flush with the outer peripheral surface 23a of the main body portion 23. The syringe tip mounting portion 25 of the present embodiment is provided so that a gap is formed between the inner peripheral surface 25b of the syringe tip mounting portion 25 and the outer peripheral surface 21b of the tip portion 21. The inner peripheral surface 25b of the syringe tip mounting portion 25 of the present embodiment is provided with a internal thread. The syringe tip mounting portion 25 of the present embodiment is formed so that the diameter of the inner peripheral surface 25b slightly decreases toward a front end side in the longitudinal direction. The tip portion 21 is provided so as to extend toward the front end side in the longitudinal direction beyond the syringe tip mounting portion 25.

The tip portion 21 of the syringe 2 of the present embodiment has a shape that can abut with the cylindrical portion of the syringe tip 3 within the syringe tip 3 described below. Specifically, an end surface 21c on the front end side in the longitudinal direction of the tip portion 21 of the syringe 2 of the present embodiment is formed so as to be able to abut with an end surface on the rear end side in the longitudinal direction of the cylindrical portion of the syringe tip 3.

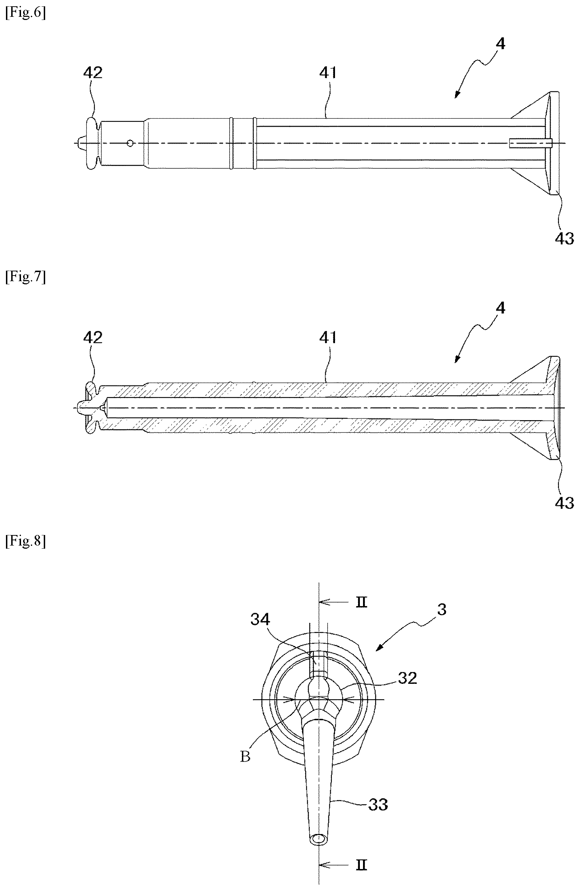

Figure 5 is a front view of the push rod 4 of the present embodiment. Figure 6 is a plan view of the push rod 4 of the present embodiment. Figure 7 is a sectional view of the push rod 4 along III-III line in Figure 5.

The push rod 4 is a member for pushing the viscous material contained in the syringe 2 toward the tip portion 21 (that is, the discharge opening 21a) of the syringe 2, and is made of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the push rod 4 is constructed from low density polyethylene.

The push rod 4 of the present embodiment has a main body portion 41 having a hollow cylindrical shape, a seal portion 42 formed at one end of the main body portion, and a finger hook portion 43 formed at the other end of the main body portion.

The main body portion 41 has a smaller outer diameter than the inner diameter of the syringe 2. Therefore, in the state that push rod 4 is contained in the syringe 2, there is a gap between the main body portion 41 and the syringe 2.

The seal portion 42 has a disc shape, and the outer peripheral end of the seal portion 42 is slidably and fluid-tightly in contact with the inner peripheral surface of the syringe 2. Although the seal portion 42 and the main body portion 41 are integrated as one member, the seal portion 42 and the main body portion 41 may be separate members.

Further, the seal portion 42 has an outer diameter larger than the inner diameter of the syringe 2 in order to fluid-tight contact the outer peripheral end with the inner peripheral surface of the syringe 2. The outer diameter of the seal portion 42 is larger than the outer diameter of the main body portion 41.

The viscous material contained in the syringe may be a dental material such as a filling composite resin, a sealant material, a cement, a bonding material, a dental splinting material, an etching material, and a tooth surface polishing material.

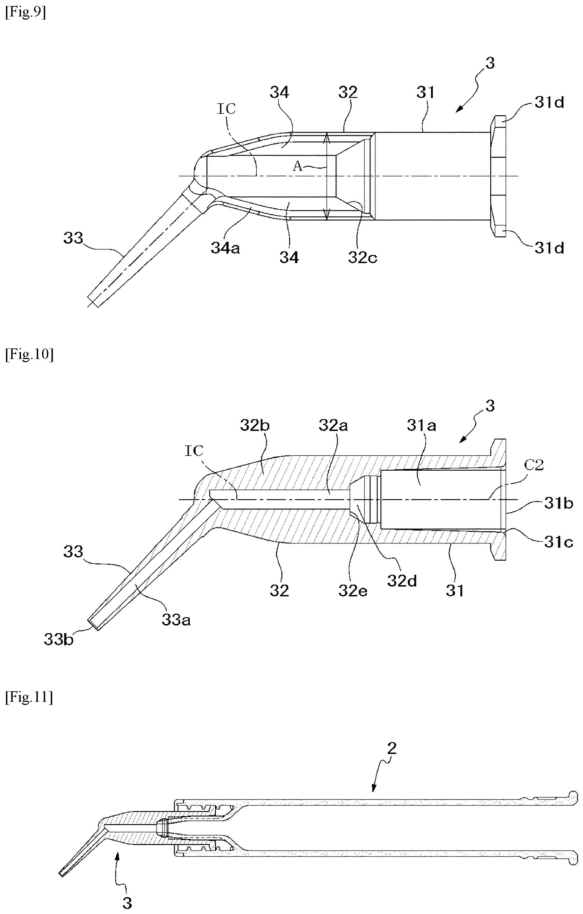

Figure 8 is a front view of a syringe tip according to one embodiment. Figure 9 is a plan view of a syringe tip according to one embodiment. Figure 10 is a sectional view along II-II line in Figure 8. Figure 11 is a sectional view of the syringe tip and a syringe in the state of attaching the syringe tip to the tip of the syringe.

The syringe tip 3 is attached to a tip of the syringe 2 containing viscous material to discharge the viscous material. The syringe tip 3 includes a syringe engaging portion 31 that engages with the syringe 2, a cylindrical portion 32 that has a hollow portion 32a having the center on the virtual central line IC and is held by the user when engaging the syringe tip 3 with the syringe 2, and a nozzle portion 33 that discharges the viscous material contained in the syringe 2.

In the present specification, the virtual central line of a hollow portion is defined as a line connecting virtual centers from the portion adjacent to the engaging portion to the portion adjacent to the nozzle in the case that the maximum feret diameter in the cross section of the hollow portion perpendicular to the nozzle direction from the engaging portion is calculated, the maximum Feret diameter is defined as the diameter, and the center of the virtual circle contacting with the cross-sectional outline of the hollow portion is defined as the virtual center.

The syringe engaging portion 31 of the present embodiment is a cylindrical member having the center on the central axis C2 and has a syringe insertion portion 31a in the inside of the syringe engaging portion 31. The tip portion of syringe 2 is inserted in the syringe insertion portion 31a. The outer diameter of the syringe engaging portion 31 may be, for example, 0.1 mm to 13 mm, and the dimension in the longitudinal direction of the syringe tip 3 may be, for example, 7 mm to 50 mm. In the present embodiment, the syringe insertion portion 31a is provided into a circular shape around the central axis C2, and may have an inner diameter of, for example, 0.1 mm to 12 mm. One end of the syringe insertion portion 31a constitutes an insertion opening 31b into which the syringe 2 is inserted. The other end of the syringe insertion portion 31a is continuous with the hollow portion 32a of the cylindrical portion 32.

In particular, the syringe insertion portion 31a of the present embodiment is formed into a tapered shape so that a cross-sectional size decreases toward the cylindrical portion 32. In the syringe insertion portion 31a of the present embodiment, in order to facilitate insertion of the syringe 2, a tapered portion 31c whose cross-sectional size slightly increases toward the insertion opening 31b is formed on a portion continuous with the insertion opening 31b.

An engagement piece 31d that is threadably attached with the internal thread of the syringe tip mounting portion 25 is projectaly mounted radially outward at the end on the the insertion opening 31b side of the outer peripheral portion of the syringe engaging portion 31 of the present embodiment. In the present embodiment, a pair (two) of engagement pieces 31d are provided facing each other in the diametrical direction, and each engagement piece 31d is constituted by a flat projecting piece in the circumferential direction. In the present invention, three or more engagement pieces 31d may be provided, and it is also possible to provide a plurality of engagement pieces 31d with their positions shifted along the central axis C2. Further, each engagement piece 31d may be formed with a somewhat spiral slope.

The syringe engaging portion 31 may be formed of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the syringe engaging portion 31 is made of polypropylene. The material of the syringe engaging portion 31 may be the same as or different from the material of the syringe 2.

The cylindrical portion 32 of the present embodiment is a cylindrical member having the center on the virtual central line IC, and has a hollow portion 32a having the center on the virtual central line IC in the inside of the cylindrical portion 32. Specifically, the cylindrical portion 32 of the present embodiment has a main body portion 32b having a cylindrical shape and being formed with a hollow portion 32a in the inside of the main body portion 32b. The outer diameter of the cylindrical portion 32 may be, for example, 0.1 mm to 13 mm, and the dimension in the longitudinal direction of the syringe tip 3 may be, for example, 0.5 mm to 5 mm. One end of the hollow portion 32a of the present embodiment is connected to the syringe insertion portion 31a of the syringe engaging portion 31 and may have an inner diameter of, for example, 0.1 mm to 12 mm. The other end of the hollow portion 32a of the present embodiment is continuous with the flow path of the viscous material formed in the inside of the nozzle portion 33. The main body portion 32b of the present embodiment is formed so that a cross-sectional size is constant. Further, the hollow portion 32a of the present embodiment is formed so that a cross-sectional size is constant.

The cylindrical portion 32 may be formed of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the cylindrical portion 32 is made of polypropylene. The material of the cylindrical portion 32 may be the same as or different from the material of the syringe 2 and the syringe engaging portion 31.

In the present embodiment, the virtual central line IC positioned at the center of the hollow portion 32a is coaxial with the central axis C1 of the syringe 2 and the central axis C2 of the syringe insertion portion 31a.

The cylindrical portion 32 of the present embodiment has a connecting portion 32c that connects the main body portion 32b and the syringe engaging portion 31. The connecting portion 32c of the present embodiment is configured to have a truncated conical shape.

The connecting portion 32c of the present embodiment is formed with a hollow portion for connection 32d that connects the hollow portion 32a and the syringe insertion portion 31a of the syringe engaging portion 31 in the inside of the connecting portion 32c. The hollow portion for connection 32d of the present embodiment is configured to have a radial dimension having the center on the virtual central line IC (hereinafter simply referred to as radial dimension) that is larger than a radial dimension of the hollow portion 32a, and is smaller than a radial dimension of the syringe insertion portion 31a. The hollow portion for connection 32d of the present embodiment has, at the end on the hollow portion 32a side, an abutted surface 32e which has an annular shape and is abutted with the end surface 21c on the front end side in the longitudinal direction of the tip portion 21 of the syringe 2.

The cylindrical portion 32 of the present embodiment is provided with a pair of blade portions 34 on the outer wall portion of the main body portion 32b. The radial dimension of the blade portion 34 may be, for example, 8 mm to 12 mm, and the dimension in the longitudinal direction of the syringe tip 3 may be, for example, 0.5 mm to 5 mm. The pair of blade portions 34 of the present embodiment are provided so as to face each other in a radial direction having the center on the virtual central line IC. The pair of blade portions 34 of the present embodiment are formed from an end on the syringe engaging portion 31 side. In other words, the pair of blade portions 34 of the present embodiment are formed over between the main body portion 32b and the connecting portion 32c. In particular, the pair of blade portions 34 of the present embodiment are formed in a manner such that the outer end in the radial direction of the pair of blade portions 34 and the outer end in the radial direction of the syringe engaging portion 31 are flush with each other. Further, the pair of blade portions 34 of the present embodiment are configured to have a reduced diameter portion 34a on the nozzle portion 33 side. The radial dimension of reduced diameter portion 34a decreases toward the nozzle portion 33.

In the syringe tip of the present embodiment, in the case that the maximum length (mm) between both end portions of the cylindrical portion on a virtual straight line orthogonal to the virtual central line is defined as first length A (see Figure 9), the virtual straight line orthogonal to the virtual central line and including both end portions of the cylindrical portion constituting the first length A is defined as first virtual straight line, and the length (mm) between both end portions of the cylindrical portion on a second virtual straight line orthogonal to the virtual central line and orthogonal to the first virtual straight line is defined as second length B (see Figure 8), the first length A and the second length B satisfy the following relational expressions (1) to (3).

$$\text{Relational expression (1): First length A / Second length B} \geq 1.5$$

Relational expression (2): $13 \geq$ (First length A + Second length B) $\geq 5$

Relational expression (3): $5 \geq$ Second length B

When the "First length A / Second length B" in Relational expression (1) is less than 1.5, a problem is caused in that sufficient blade portion required for applying force during tightening cannot be secured or that the surgical field is obstructed. The "First length A / Second length B" may be 12 or less. When the "First length A / Second length B" is set to 12 or less, the effect that operability is good in performing filling work in the oral cavity, and that rigidity can be ensured during installation so that tightening can be performed reliably is exhibited.

When the "First length A + Second length B" in the Relational expression (2) is less than 5, a problem is caused in that the strength of the cylindrical portion is insufficient and tightening is insufficient due to difficult to grip. When the "First length A + Second length B" in the Relational expression (2) is larger than 13, a problem is caused in that the surgical field is obstructed and operability is worsened in performing filling work in the oral cavity because the cylindrical portion is too large.

When the second length B in the Relational expression (3) is larger than 5, a problem is caused in that the surgical field is obstructed and operability is worsened in performing filling work in the oral cavity because the cylindrical portion is too large. The second length B may be 0.5 or more. When the second length B is set to 0.5 or more, the effect that the rigidity of the cylindrical portion can be ensured, and therefore tightening can be performed reliably without deformation during installation is exhibited.

In the above embodiment, the cylindrical portion 32 includes the main body portion 32b formed with the hollow portion 32a, and the pair of blade portions 34 provided on the outer wall portion of the main body portion 32b. In such a case, the first length A may be the length between the tip portions of the pair of blade portions, and the second length B may be the length between the outer walls of the main body portion 32b. In the above embodiment, the length between the tip portions of the pair of blade portions, that is the first length A, is 5.6 mm, and the length between the outer walls of the main body portion 32b, that is the second length B, is 2.3 mm.

The first length A may satisfy the following relational expression (4).

Relational expression (4): First length A $\leq 8$

When the first length A in the Relational expression (4) is 8 or less, an effect that visibility can be improved is exhibited. Further, the first length A may be 5 or less. When the first length A is 5 or less, the effect that visibility is further improved is exhibited.

The nozzle portion 33 discharges the viscous material contained in the syringe 2. Specifically, the nozzle portion 33 of the present embodiment is formed with a flow path 33a of viscous material in the inside of the nozzle portion 33, and one end of the flow path 33a forms a discharge opening 33b. A dimension of the nozzle portion 33 in the longitudinal direction of the syringe tip 3 may be, for example, 1 mm to 40 mm. Further, the dimension of the nozzle portion 33 in a direction in which the flow path 33a extends may be 5 mm or more, and may be 8 mm or more. By setting the dimension of the nozzle portion in a direction in which the flow path 33a extends to such a length, it is possible to easily fill the molars which are at the innermost portion of the oral cavity with the viscous material, for example.

In the present invention, in the case that an inner diameter (mm) of the nozzle portion on the discharge opening side is defined as inner diameter C, the inner diameter C may satisfy the following relational expression (5).

Relational expression (5): $1.50 \geq$ Inner diameter C $\geq 0.12$

When the inner diameter C in the Relational expression (5) is less than 0.12, there is a case that a problem is caused in that it is difficult to discharge paste. When the inner diameter C in the Relational expression (5) is larger than 1.5, there is a case that a problem is caused in that the discharge amount of paste becomes larger than the intended amount, and filling becomes difficult.

In this specification, the inner diameter of the nozzle portion on the discharge opening side means the minimum diameter of the nozzle tip potrion.

The nozzle portion 33 of the present embodiment is formed in a manner such that the cross-sectional size decreases toward the discharge opening 33b. Further, the flow path 33a of the present embodiment is formed in a manner such that the cross-sectional size decreases toward the discharge opening 33b.

The nozzle portion 33 may be formed of, for example, a resin material or a metal material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the nozzle portion 33 is made of polypropylene.

In the syringe tip of the present embodiment, the nozzle portion, the cylindrical portion and the syringe engaging portion are integrally formed.

In the present embodiment, in the case that the outer diameter (mm) of the main body portion 32b of the cylindrical portion 32 is defined as D32b and the outer diameter (mm) of the syringe 2 is defined as D2, the outer diameter D32b and the outer diameter D2 may satisfy the following relational expression (6) and may satisfy the following relational expression (7).

$$\text{Relational expression (6): Outer diameter } D2 \times 0.5 \geq \text{Outer diameter } D32b$$

$$\text{Relational expression (7): Outer diameter } D2 \times 0.3 \geq \text{Outer diameter } D32b$$

By satisfying such a relational expression, it is possible to obtain the effect that the surgical field is wider and the operability in the oral cavity is superior.

In the present embodiment, in the case that the inner diameter (mm) on the discharge opening side of the nozzle portion is defined as C and the outer diameter (mm) of the main body portion 32b of the cylindrical portion 32 is defined as D32b, the inner diameter C and the outer diameter D32b may satisfy the following relational expression (8).

Relational expression (8): Outer diameter D32b $\geq$ Inner diameter C

By satisfying such a relational expression, it is possible to obtain the effect that the surgical field is wider and the operability in the oral cavity is superior.

In the present embodiment, in the case of viewing from the longitudinal direction of the syringe tip, the angle between the plane on which the pair of blade portions extend and the plane on which the nozzle portion extends may be 45° or less.

By setting such an angle range, it is possible to obtain the effect that the surgical field is wider and the operability in the oral cavity is superior.

In the present embodiment, the First length A and the outer diameter D2 of the syringe 2 may satisfy the following relational expression (9).

$$\text{Relational expression (9): Outer diameter } D2 \times 0.9 \geq \text{First length } A$$

By satisfying such a relational expression, it is possible to obtain the effect that the surgical field is wider and the operability in the oral cavity is superior.

In the above embodiment, the tip portion of the syringe is configured to be able to abut with the syringe tip within the syringe tip. Therefore, it is possible to suppress the inclusion of air bubbles when the viscous material moves from the syringe to the syringe tip, and it is also possible to reduce the amount of viscous material remaining in the syringe tip and it is possible to suppress the viscous material from dripping from the syringe in the case of replacing the syringe tip.

In the syringe tip of the above embodiment, the nozzle portion, the cylindrical portion and the syringe engaging portion are integrally formed from a resin material. Therefore, press-fitting of the nozzle is not required during manufacturing, and it is possible to facilitate manufacturing. Further, since the nozzle portion, the cylindrical portion and the syringe engaging portion are made of only resin material, there is no need to separate metals at the time of disposal, and disposal can be easily performed.

In the above embodiment, a dental material is used as the viscous material, but pharmaceuticals, medical materials, cosmetics, foods and the like may be used as the viscous material.

In the above embodiment, the cylindrical portion comprises a main body portion formed with a hollow portion and a pair of blade portions provided on the outer wall portion of the main body portion. However, the syringe tip of the present invention may not have a blade portion. Further, in the above embodiment, two blade portions are provided, however the number of blade portion is not limited to two, and may be one, or three or more.

In the above embodiments, the syringe tip has a symmetrical shape, but the syringe tip may have an asymmetrical shape.

The syringe tip of the above embodiment is configured in a manner such that an extending direction of the nozzle portion is bent from an extending direction of the cylindrical portion, however, the nozzle portion and the cylindrical portion may be configured linearly.

In the syringe tip of the above embodiment, the flow path of the nozzle portion is formed linearly, however, the flow path of the nozzle portion may be formed in a curved shape.

In the syringe tip of the above embodiment, the nozzle portion, the cylindrical portion and the syringe engaging portion are integrally formed, however, the nozzle portion, the cylindrical portion and the syringe engaging portion may be individually formed as separate members.

In the syringe tip of the above embodiment, the nozzle portion is made of a resin material, however the nozzle portion may be made of a metal material.

In the syringe tip of the above embodiment, the nozzle portion 33 is formed in a manner such that the cross-sectional size decreases toward the discharge opening 33b, however, the cross-sectional size of the nozzle portion 33 may be constant.

In the syringe tip of the above embodiment, the flow path 33a is formed in a manner such that the cross-sectional size decreases toward the discharge opening 33b, however, the cross-sectional size of the flow path 33a may be constant.

In the above embodiment, the syringe tip and the syringe are threadably attached together using an internal thread type, but the syringe tip and the syringe may also be threadably attached together using a so-called external thread type.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention can provide a syringe tip that can be securely attached to the syringe and can provide excellent operability without obstructing the surgical field, and a set of the syringe tip and a syringe.

Reference Signs List

1: injector
2: syringe
21: tip portion
21a: discharge opening
21b: outer peripheral surface
21c: end surface
22: rear end portion
22a: insertion opening
22b: finger grip attachment portion
22c: flange portion
22d: flange portion
22e: recess portion
23: main body portion
23a: outer peripheral surface
24: hollow portion
24a: first tapered portion
24b: first columnar portion
24c: second tapered portion
24d: second columnar portion
24e: third tapered portion

25: syringe tip mounting portion
25a: outer peripheral surface
25b: inner peripheral surface
3: syringe tip
31: syringe engaging portion
31a: syringe insertion portion
31b insertion opening
31c: tapered portion
31d: engagement piece
32: cylindrical portion
32a: hollow portion
32b: main body portion
32c: connecting portion
32d: hollow portion for connection
32e: abutted surface
33: nozzle portion
34: blade portion
34a: reduced diameter portion
4: push rod
5: finger grip
C1: central axis
C2: central axis
IC: virtual central line

**Claims**

1. A syringe tip to be attached to a tip of a syringe containing viscous material to discharge the viscous material, comprising

   a syringe engaging portion that engages with the syringe,
   a cylindrical portion having a hollow portion with a virtual center line at its center and held by a user in the case of engaging the syringe tip with the syringe, and
   a nozzle portion discharging the viscous material contained in the syringe,
   wherein in the case that the maximum length (mm) between both end portions of the cylindrical portion on a virtual straight line orthogonal to the virtual central line is defined as first length A, the virtual straight line orthogonal to the virtual central line and including both end portions of the cylindrical portion constituting the first length A is defined as first virtual straight line, and the length (mm) between both end portions of the cylindrical portion on a second virtual straight line orthogonal to the virtual central line and orthogonal to the first virtual straight line is defined as second length B,
   the first length A and the second length B satisfy the following relational expressions (1) to (3).

$$\text{Relational expression (1): First length A / Second length B} \geq 1.5$$

$$\text{Relational expression (2): } 13 \geq (\text{First length A} + \text{Second length B}) \geq 5$$

$$\text{Relational expression (3): } 5 \geq \text{Second length B}$$

2. The syringe tip according to claim 1, wherein

   the cylindrical portion comprises a main body portion formed with the hollow portion and a pair of blade portions provided on an outer wall portion of the main body portion,
   the first length A is a length between the tip portions of the pair of blade portions, and
   the second length B is a length between the outer walls of the main body portion.

3. The syringe tip according to claim 1 or 2, wherein

the nozzle portion extends in a direction inclined with respect to the virtual central line of the hollow portion of the cylindrical portion, and
the pair of blade portions are provided parallel to an extending direction of the nozzle portion.

4. The syringe tip according to any one of claims 1 to 3, wherein
the First length A further satisfies the following relational expression (4).

$$\text{Relational expression (4): First length } A \leq 8$$

5. The syringe tip according to any one of claims 1 to 4, wherein

in the case that an inner diameter (mm) of the nozzle portion on the discharge opening side is defined as inner diameter C,
the inner diameter C satisfies the following relational expression (5).

$$\text{Relational expression (5): } 1.50 \geq \text{Inner diameter } C \geq 0.12$$

[Fig.1]

3

1

2

5

4

[Fig.2]

I

2

21a

I

[Fig.3]

[Fig.4]

[Fig.5]

[Fig.6]

42    41    4

43

[Fig.7]

42    41    4

43

[Fig.8]

II

34    3

32

B

33

II

[Fig.9]

[Fig.10]

[Fig.11]

Europäisches
Patentamt

European
Patent Office

Office européen
des brevets

# EUROPEAN SEARCH REPORT

Application Number

EP 23 19 9270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 6 282441 B2 (TAISEI KAKO CO) 21 February 2018 (2018-02-21) * paragraphs [0004] - [0006], [0019] - [0020]; figures 2,6 * | 1-5 | INV. B05C17/005 |
| A | JP 2007 006994 A (TAISEI KAKO CO; SHOFU KK) 18 January 2007 (2007-01-18) * paragraphs [0013] - [0016]; figures 4-8 * | 1-5 | |
| A | US 2018/008513 A1 (IIBUCHI RURIKO [JP] ET AL) 11 January 2018 (2018-01-11) * paragraphs [0002], [0050] - [0054]; figures 1,2,7 * | 1-5 | |
| A | US 2013/112720 A1 (CROSS CHRISTOPHER [US]) 9 May 2013 (2013-05-09) * paragraphs [0004], [0022], [0024]; figures 1,2 * | 1-5 | |
| A | JP 2012 148070 A (SUN MEDICAL CO LTD) 9 August 2012 (2012-08-09) * paragraphs [0001], [0006]; figure 1 * | 1,5 | TECHNICAL FIELDS SEARCHED (IPC) B05C A61C |

The present search report has been drawn up for all claims

| Place of search | Date of completion of the search | Examiner |
|---|---|---|
| The Hague | 6 March 2024 | Blazquez Lainez, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 9270

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2024

| Patent document cited in search report | | | Publication date | Patent family member(s) | | | Publication date |
|---|---|---|---|---|---|---|---|
| JP | 6282441 | B2 | 21-02-2018 | JP | 6282441 | B2 | 21-02-2018 |
| | | | | JP | 2015089420 | A | 11-05-2015 |
| JP | 2007006994 | A | 18-01-2007 | JP | 4658710 | B2 | 23-03-2011 |
| | | | | JP | 2007006994 | A | 18-01-2007 |
| US | 2018008513 | A1 | 11-01-2018 | EP | 3275477 | A1 | 31-01-2018 |
| | | | | JP | WO2016153003 | A1 | 25-01-2018 |
| | | | | US | 2018008513 | A1 | 11-01-2018 |
| | | | | WO | 2016153003 | A1 | 29-09-2016 |
| US | 2013112720 | A1 | 09-05-2013 | NONE | | | |
| JP | 2012148070 | A | 09-08-2012 | NONE | | | |

EPO FORM P0459

For more details about this annex : see Official Journal of the European Patent Office, No. 12/82

**EP 4 357 030 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5445523 A